# EUROPEAN PATENT APPLICATION

(11) **EP 4 019 052 A1**
(43) Date of publication of application: **29.06.2022**
(21) Application number: 21191847.9
(22) Date of filing: 18.08.2021
(51) Int. Cl.: A61L 2/10, A61L 2/24, A61L 2/28, B64F 5/30, G06T 19/00

(54) **DISINFECTION DEVICES, SYSTEMS, AND MATERIALS**

(30) Priority: 03.09.2020 US 202063074042 P; 08.07.2021 US 202117370376
(71) Applicant: B/E Aerospace, Inc., Winston-Salem, NC 27105 (US)
(72) Inventor: EDQUIST, John, Milwaukee, 53207 (US); PHIFER, Van, Greendale, 53129 (US); JOHANNESSEN, Eric, Holbrook, 11741 (US)
(74) Representative: Dehns

(57) **Abstract**

A handheld disinfection device (110) includes a housing (112), an electromagnetic radiation source (114), a sensor (116), and a controller (118). The housing (112) is configured to be grasped by an operator (60) and the electromagnetic radiation source (114) is affixed to the housing (112) and is configured to operably emit disinfecting electromagnetic radiation (115). The sensor (116) and the controller (118) are coupled to the housing (112), and the controller (118) includes a processor and a memory having instructions stored thereon that cause the processor to perform various operations. The various operations may include receiving, by the processor, operational data from the sensor (116) pertaining to an operator's manipulation of the handheld disinfection device (110) during a disinfecting treatment. The controller (118) operations may further include determining, by the processor, operational feedback based on the operational data, wherein the operational feedback comprises information pertaining to whether a proper dosage of disinfecting electromagnetic radiation (115) has been directed to a surface of a structure to be disinfected.

## Description

### CROSS-REFERENCE TO RELATED APPLICATIONS

This application claims priority to, and the benefit of, U.S. Provisional Application Serial No. 63/074,042, filed September 3, 2020 and titled " DISINFECTION DEVICES, SYSTEMS, AND MATERIALS".

### FIELD

The present disclosure relates to devices, systems, and methods for pathogen disinfection, and in particular to improving efficiency and effectiveness of disinfection treatments.

### BACKGROUND

The recent novel-coronavirus (SARS-COV-2) outbreak has negatively impacted the safety and health of many people. In situations where different users occupy the same space at different times, there is a risk of indirect contact transmission of pathogens. For example, lingering pathogens may remain on contact surfaces of an aircraft cabin to be spread to passengers and/or crew members on a subsequent flight. The safety of such passengers and crew members may be improved by treating surfaces, such as seats, ceiling/wall panels, handles, and lavatory surfaces, etc., with disinfecting treatments capable of mitigating the presence of pathogens on such surfaces. However, the quality and effectiveness of such treatments are difficult to verify and/or track. Further, certain treatments, such as ultraviolet radiation treatments, may result in harm to the operator if not properly effectuated.

### SUMMARY

In various embodiments, the present disclosure provides a handheld disinfection device that includes a housing, an electromagnetic radiation source, a sensor, and a controller. The housing may be configured to be grasped by an operator, and the electromagnetic radiation source may be affixed to the housing and may be configured to operably emit disinfecting electromagnetic radiation. The sensor and the controller may be coupled to the housing, and the controller may include a processor and a tangible, non-transitory computer-readable storage medium (i.e., a memory) having instructions stored thereon that, in response to execution by the processor, cause the processor to perform various operations. The various operations may include receiving, by the processor, operational data from the sensor pertaining to an operator's manipulation of the handheld disinfection device during a disinfecting treatment. The controller operations may further include determining, by the processor, operational feedback based on the operational data, wherein the operational feedback comprises information pertaining to whether a proper dosage of disinfecting electromagnetic radiation has been directed to a surface of a structure to be disinfected.

In various embodiments, the operations further comprise providing, by the processor, the operational feedback to the operator. For example, the handheld disinfection device may include a display screen configured to display at least one of the operational data and the operational feedback for the operator. In various embodiments, the operational feedback is dynamic operational feedback such that determining and providing, by the processor, the dynamic operational feedback to the operator is performed substantially in real-time. The handheld disinfection device may further include an indicator coupled to the housing, wherein the indicator is configured to provide at least one of visible, audible, and haptic indications to the operator representative of the dynamic operational feedback.

In various embodiments, determining, by the processor, the operational feedback comprises tracking, by the processor, dosage of the disinfecting electromagnetic radiation. Tracking the dosage of the disinfecting electromagnetic radiation comprises mapping the disinfecting electromagnetic radiation across the surface of the structure that is susceptible to indirect contact transmission of pathogens, according to various embodiments. The handheld disinfection device may further include a wireless connection module affixed to the housing, wherein the operations comprise detecting, by the processor, a dynamic location of the handheld disinfection device. The controller operations may further include actively modulating, by the processor, the disinfecting electromagnetic radiation emitted from the electromagnetic radiation source based on the operational data.

The sensor may include at least one of an accelerometer, a proximity sensor, a location sensor, and an atmospheric sensor. For example, the sensor may include an atmospheric sensor configured to detect humidity of air around the handheld disinfection device. The handheld disinfection device may further include a targeting module coupled to the housing and configured to emit visible electromagnetic radiation indicative of the disinfecting electromagnetic radiation emitted from the electromagnetic radiation source.

Also disclosed herein, according to various embodiments, is a disinfection system that includes a handheld disinfection device and a wearable. The handheld disinfection device may include an electromagnetic radiation source configured to operably emit disinfecting electromagnetic radiation. The wearable may be configured to be worn by an operator, wherein the wearable is configured to be coupled in electric communication with the handheld disinfection device. The wearable may be configured to provide operational feedback to the operator, wherein the operational feedback comprises information pertaining to whether a proper dosage of the disinfecting electromagnetic radiation has been directed to a surface of a structure to be disinfected.

In various embodiments, the operational feedback is dynamic. The wearable may be glasses configured to visually display the operational feedback substantially in real-time to the operator. The glasses may include physical filters configured to enable the operator to see at least one of a presence and intensity of the disinfecting electromagnetic radiation. In various embodiments, the glasses comprise one or more augmented reality screens configured to enable the operator to see calculated digital depictions of at least one of a presence and intensity of the disinfecting electromagnetic radiation.

Also disclosed herein, according to various embodiments, is an article of manufacture that includes a structure comprising a surface susceptible to indirect contact transmission of pathogens and a material at least one of embedded within, coupled to, and integrated with the surface of the structure, with the material being configured to provide feedback to an operator pertaining to a disinfecting dosage experienced by the surface in response to a disinfecting treatment. In various embodiments, the structure is an aircraft seat of an aircraft, and the material comprises an array of nodes distributed across the surface of the aircraft seat. The disinfecting treatment comprises disinfecting electromagnetic radiation, wherein the array of nodes comprise photoluminescence units configured to luminesce in response to photoexcitation from the disinfecting treatment, according to various embodiments.

The foregoing features and elements may be combined in various combinations without exclusivity, unless expressly indicated herein otherwise. These features and elements as well as the operation of the disclosed embodiments will become more apparent in light of the following description and accompanying drawings.

### BRIEF DESCRIPTION OF THE DRAWINGS

FIG. 1 illustrates a view of a cabin of an aircraft, in accordance with various embodiments;
FIG. 2 illustrates a perspective view of a handheld disinfection device, in accordance with various embodiments;
FIG. 3A is a schematic flow chart diagram showing processes of a controller of a handheld disinfection device, in accordance with various embodiments;
FIG. 3B is a schematic flow chart diagram showing processes of a controller of a handheld disinfection device, in accordance with various embodiments;
FIG. 3C is a schematic flow chart diagram showing processes of a controller of a handheld disinfection device, in accordance with various embodiments;
FIG. 4 illustrates a perspective view of a disinfection system having a handheld disinfection device and a wearable, in accordance with various embodiments;
FIG. 5 illustrates a perspective view of a handheld disinfection device, in accordance with various embodiments; and
FIG. 6 illustrates a perspective view of an article of manufacture having a material configured to provide feedback to an operator of a handheld disinfection device, in accordance with various embodiments.

The subject matter of the present disclosure is particularly pointed out and distinctly claimed in the concluding portion of the specification. A more complete understanding of the present disclosure, however, may best be obtained by referring to the detailed description and claims when considered in connection with the drawing figures.

### DETAILED DESCRIPTION

The detailed description of exemplary embodiments herein makes reference to the accompanying drawings, which show exemplary embodiments by way of illustration. While these exemplary embodiments are described in sufficient detail to enable those skilled in the art to practice the disclosure, it should be understood that other embodiments may be realized and that logical changes and adaptations in design and construction may be made in accordance with this disclosure and the teachings herein without departing from the spirit and scope of the disclosure. Thus, the detailed description herein is presented for purposes of illustration only and not of limitation.

Disclosed herein, according to various embodiments, are devices, systems, methods, and articles of manufacture for effectuating a disinfecting treatment of a surface that is susceptible to indirect contact transmission of pathogens. Generally, the devices, systems, methods, and articles of manufacture disclosed and described herein facilitate the quality, repeatability, and/or overall effectiveness of a disinfection treatment, according to various embodiments. Said differently, the present disclosure may generally pertain to providing feedback to an operator tasked with disinfecting various components and structures, such as aircraft cabin surfaces. By improving the feedback to the operator during a disinfection treatment, a more thorough sanitation can be performed, thereby helping to improve the health and safety of passengers and crew members. Although numerous details and examples are included herein pertaining to utilizing these concepts to aircraft cabins, the present disclosure is not necessarily so limited, and thus aspects of the disclosed embodiments may be adapted for performance in a variety of other industries (e.g., trains, vehicles, buildings, hotels, etc.). As such, numerous applications of the present disclosure may be realized. Further, while many details herein specifically refer to ultraviolet-type disinfecting devices, the general details of the disclosure may be implemented with other types of disinfecting devices, such as antimicrobial spray devices.

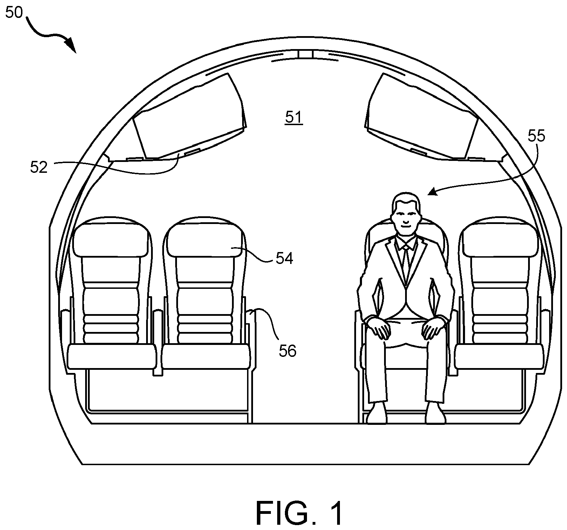

With reference to FIG. 1, a cabin 51 of an aircraft 50 is shown, according to various embodiments. The aircraft 50 may be any aircraft such as an airplane, a helicopter, or any other aircraft. Pathogens, such as viruses and bacteria, may remain on surfaces of the cabin 51, and these remaining pathogens may result in indirect contact transmission to other people (e.g., subsequent passengers). For example, the cabin 51 may include overhead bins 52, passenger seats 54 for supporting passengers 55, handles 56, lavatory surfaces, and other structures/surfaces upon which active pathogens may temporarily reside. As will be discussed further below, in order to reduce the transmission/transfer or pathogens between passengers, one or more operators may perform a disinfecting treatment to the cabin 51 between flights.

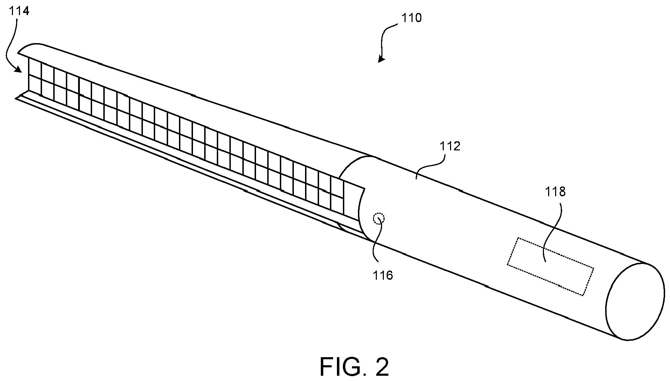

In various embodiments, and with reference to FIG. 2, a handheld disinfection device 110 is provided. The handheld disinfection device 110, according to various embodiments, includes a housing 112 (e.g., a body or structural casing) and an electromagnetic radiation source 114 affixed to the housing 112. Generally, the electromagnetic radiation source 114 is configured to operably emit disinfecting electromagnetic radiation, according to various embodiments. For example, the electromagnetic radiation source 114 may be configured to emit short wavelength light, such as ultraviolet light (commonly referred to as "ultraviolet C" or "UV-C") to kill or at least inactivate pathogens, as described in greater detail below. In various embodiments, the disinfecting electromagnetic radiation is 'far' UV-C radiation, such as electromagnetic radiation having a wavelength of about 222 nm. As used herein, the term "about" means plus or minus 5 nm. The handheld disinfection device 110 may further include a sensor 116 coupled to the housing 112. In various embodiments, the handheld disinfection device 110 includes multiple sensors. The handheld disinfection device 110 further includes a controller 118 coupled to the housing, with the controller comprising a processor and a tangible, non-transitory computer-readable storage medium (i.e., a memory) having instructions stored thereon that, in response to execution by the processor, cause the processor to perform various operations, according to various embodiments. The operations may include receiving, by the processor, operational data from the sensor 116 pertaining to an operator's manipulation of the handheld disinfection device during a disinfecting treatment. The components, functionality, operation, and some benefits of the handheld disinfection device 110 are described in more detail in the following paragraphs.

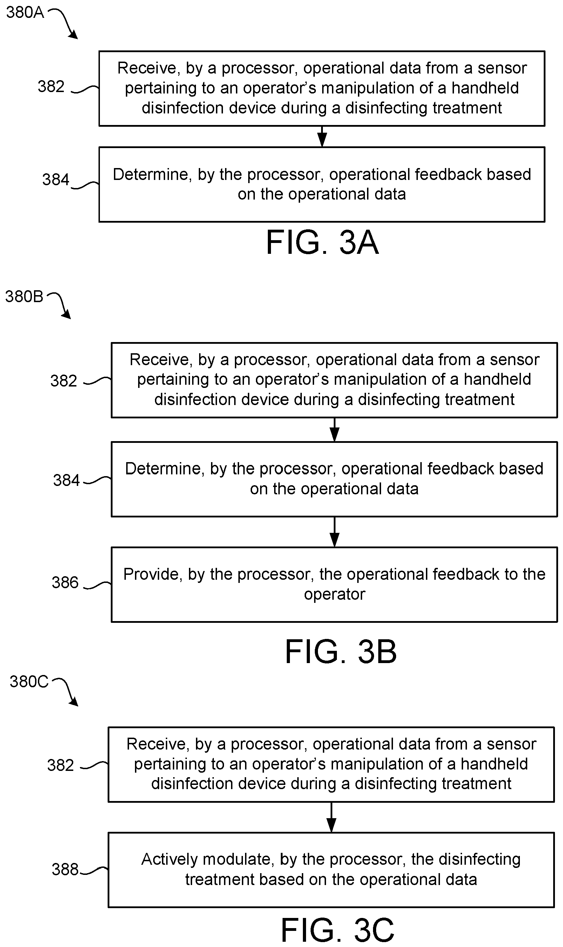

The handheld disinfection device 110 is generally configured to provide various benefits over conventional sanitation devices. In various embodiments, the operational data received by the controller 118 from the sensor 116 is utilized to improve the effectiveness of disinfecting treatments. For example, and as described in greater detail below with reference to FIGS. 3A, the processor of the controller 118 may be configured to determine operational feedback based on the detected operational data from the sensor 116. This operational feedback may be provided, via the processor of the controller 118, to the operator of the handheld disinfection device 110 to allow the operator to adjust how to use the handheld disinfection device 110 in order to improve the effectiveness of the disinfecting treatment (e.g., see FIG. 3B, as described in greater detail below). In various embodiments, the operational data received from the sensor 116 may be utilized to actively/directly modulate the disinfecting treatment (e.g., see FIG. 3C, as described in greater detail below). Thus, the inclusion of the sensor 116 electrically connected to the controller 118 generally facilitates improved operation of the handheld disinfection device 110, thereby resulting in improved pathogen disinfection and improved health and safety, according to various embodiments. Further, the handheld disinfection device 110 may help to prevent the operator from inadvertently causing harm to himself via improper use of the device.

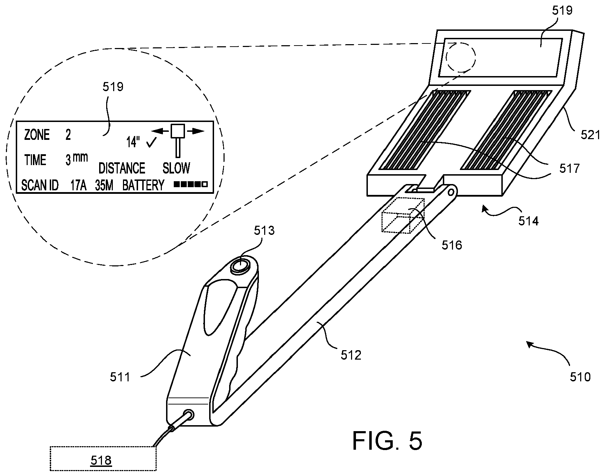

Returning to the components of the handheld disinfection device 110, the housing 112 is the body/casing that provides the structure of the handheld disinfection device 110. The housing 112 may be made from a plastic material, such as a thermoset or thermoplastic material. The housing may include a handle portion to allow the operator to easily and ergonomically grasp the handheld disinfection device. The housing may include a telescoping or otherwise extendable structure to enable the operator to properly position the electromagnetic radiation source in proximity to higher surfaces, such as over-head bins, during the disinfection treatment. The housing may further include a body portion and a head portion having the electromagnetic radiation source affixed thereto, wherein the head portion is pivotably connected to the body portion to enable the operator to select a desired orientation of the electromagnetic radiation source. Additional details pertaining to the housing and the handheld disinfection device in general are included below with reference to FIG. 5.

In various embodiments, the electromagnetic radiation source 114 includes multiple light emitters forming an array or a pattern of electromagnetic radiation sources. The electromagnetic radiation source may include one or more light emitting diodes ("LEDs"). In various embodiments, the electromagnetic radiation source provides the disinfecting electromagnetic radiation from an incandescent source, an excimer lamp, a gas discharge source, a laser-type source, or other electromagnetic radiation sources. As mentioned above, the electromagnetic radiation source is generally configured to emit UV-C light. UV-C light may effectively deactivate various pathogens, such as bacteria, viruses, fungal spores, and other microorganisms. The UV-C light may have a wavelength of between about 200 nanometers and about 300 nanometers. As used in this context only, the term "about" refers to plus or minus 10 nanometers.

In various embodiments, the sensor 116 is configured to detect a condition or parameter that affects the disinfecting treatment. The sensor 116 may be a plurality of sensors (e.g., the handheld disinfection device 110 may include multiple sensors). The sensor 116 in FIG. 2 is shown schematically, and thus the number, location, and/or size of the sensor(s) may be different than what is depicted in FIG. 2. In various embodiments, the sensor 116 includes an accelerometer, a proximity sensor, a location sensor, a radio frequency identification reader, a camera, and/or an atmospheric sensor. An accelerometer may detect orientation and/or movement of the handheld disinfection device relative to the target surface to be sanitized. As used herein, the term "target surface" refers to a surface of a structure or component that is susceptible to indirect contact transmission of pathogens. In various embodiments, a proximity sensor detects a distance between the electromagnetic radiation source and the target surface. A location sensor may detect a position/location of the handheld disinfection device relative to an overall system, such as an aircraft cabin. An atmospheric sensor may detect the atmospheric/environmental conditions of the air through which the electromagnetic radiation propagates from the electromagnetic radiation source to the target surface. For example, the atmospheric sensor may detect humidity of the air around the handheld disinfection device, which can affect the transmission of the electromagnetic radiation. Additional details pertaining to these sensors and the detected operational data they can provide to the controller 118 are provided below with reference to FIGS. 3A, 3B, and 3C.

In various embodiments, the controller 118 is a standalone controller that is affixed/integrated into the housing 112 of the handheld disinfection device 110. The controller 118 in FIG. 2 is shown schematically, and thus the size, position, and orientation of the controller may be different than what is depicted in FIG. 2. In various embodiments, the controller may be integrated into computer systems onboard an aircraft. In various embodiments, the controller 118 comprises a processor. In various embodiments, the controller 118 is implemented in a single processor. In various embodiments, the controller 118 may be implemented as and may include one or more processors and/or one or more tangible, non-transitory memories and be capable of implementing logic. Each processor can be a general purpose processor, a digital signal processor (DSP), an application specific integrated circuit (ASIC), a field programmable gate array (FPGA) or other programmable logic device, discrete gate or transistor logic, discrete hardware components, or any combination thereof. The controller 118 may comprise a processor configured to implement various logical operations in response to execution of instructions, for example, instructions stored on a non-transitory, tangible, computer-readable medium configured to communicate with the controller 118.

System program instructions and/or controller instructions may be loaded onto a non-transitory, tangible computer-readable medium having instructions stored thereon that, in response to execution by the processor, cause the controller to perform various operations. The term "non-transitory" is to be understood to remove only propagating transitory signals per se from the claim scope and does not relinquish rights to all standard computer-readable media that are not only propagating transitory signals per se. Stated another way, the meaning of the term "non-transitory computer-readable medium" and "non-transitory computer-readable storage medium" should be construed to exclude only those types of transitory computer-readable media which were found in In Re Nuijten to fall outside the scope of patentable subject matter under 35 U.S.C. § 101.

The instructions stored on the memory of the controller 118 may be generally configured to perform various operations. The schematic flow chart diagrams of FIGS. 3A, 3B, and 3C include various exemplary methods 380A, 380B, 380C that the processor of the controller 118 may perform. In various embodiments, and with reference to FIG. 3A, the controller method 380A may include receiving, by the processor, operational data (e.g., detected data) from a sensor 116 at step 382. The operational data received at step 382 may include information pertaining to an operator's manipulation of the handheld disinfection device during a disinfecting treatment. For example, as introduced above, an accelerometer may provide operational data pertaining to orientation and/or speed of the handheld disinfection device, a proximity sensor may provide operational data pertaining to a distance between the electromagnetic radiation source and the target surface, a location sensor may provide operational data pertaining to a position/location of the handheld disinfection device relative to an overall system, such as an aircraft cabin, and an atmospheric sensor may provide operational data pertaining to atmospheric/environmental conditions of the air through which the electromagnetic radiation propagates from the electromagnetic radiation source to the target surface.

In various embodiments, controller method 380A includes determining, by the processor, operational feedback based on the operational data at step 384. The operational feedback may comprise information pertaining to whether a proper dosage of disinfecting electromagnetic radiation has been directed to a surface of a structure to be disinfected (i.e., the target surface). That is, the detected data from the one or more sensors may be analyzed/interpreted into operational feedback that is useful for improving the quality and/or effectiveness of disinfecting treatments. The operational feedback may be stored/tracked to allow for the operator to subsequently review the feedback in order to glean information on how to use/manipulate the device in order to improve the desired disinfection results. In various embodiments, the operational feedback may be transmitted to the operator's trainer (or training agency) and/or to the manufacturer of the handheld disinfection device to enable adjustments to be made in order to improve the capability of the operator to effectively perform the disinfecting treatment.

In various embodiments, and with reference to FIG. 3B, controller method 380B further includes providing, by the processor, the operational feedback data to the operator at step 386. This step may be performed substantially in real-time. That is, the operational data and/or the operational feedback is dynamic, and is thus indicative of the live/actual operating condition of the device. By providing real-time feedback to the operator, the operator may be able to actively adjust his or her manipulation of the handheld disinfection device. For example, step 386 may include alerting the operator if he/she is improperly orientating the electromagnetic radiation source relative to the target surface, if he/she is moving too fast or slow across the target surface, or if the operator has skipped over certain areas (e.g., via the location sensor). In various embodiments, step 386 may include notifying the operator of the overall dosage of disinfecting electromagnetic radiation applied to the target surface, thereby not only helping to ensure the entire target surface is disinfected but also potentially enabling the operator to move more efficiently, as there may be less time wasted disinfecting an area that has already been sufficiently sanitized.

In various embodiments, the controller method may include tracking, by the processor, dosage of the disinfecting electromagnetic radiation. In various embodiments, this dosage tracking may include mapping the disinfecting electromagnetic radiation across the target surface of the structure (i.e., across the surface being disinfected that is susceptible to indirect contact transmission of pathogens). This location mapping may be achieved by the sensor including location tracking relative to the overall system. For example, the sensor may include a wireless connection module affixed to the housing, and the system (such as an aircraft cabin) may include a wireless mesh network (e.g., a Bluetooth mesh network). Thus, the wireless connection module may enable the controller operations to detect and/or track the dynamic location of the handheld disinfection device. In various embodiments, the sensor may include a camera or other optical scanning mechanism, and the target surface to be disinfected may include discrete location markers (e.g., fiducials) that can be detected by the camera/scanning mechanism. For example, the target surface may include an array of RFID tags or QR codes that can be sensed by an RFID reader or a camera, respectively, to facilitate tracking/mapping the movement of the handheld disinfection device.

In various embodiments, and with momentary reference to FIG. 5, the handheld disinfection device 510 includes a display screen 519 or other indicator configured to display at least one of the operational data and the operational feedback for the operator. That is, the display screen may provide information pertaining to the disinfecting treatment, such as scanning speed, location, distance from the target surface, and battery level, among other items of information. In various embodiments, the housing may include an indicator coupled to the housing 512, such as the display screen 519, configured to provide at least one of visible, audible, and/or haptic indications to the operator representative of the operational data and/or the dynamic operational feedback. For example, the indicator may provide light, sound, and/or vibration-type alerts to actively guide the operator's manipulation of the handheld disinfection device. In various embodiments, the operational feedback determined by the processor at step 384 may not only provide the benefit of improved sanitation of the target structures/surfaces, but the operational feedback may allow for untrained or minimally trained operators to properly wield the handheld disinfection device to successfully perform an effective disinfection treatment.

In various embodiments, and with reference to FIG. 3C, the controller method 380C includes actively modulating, by the processor, the disinfecting treatment based on the operational data at step 388. That is, instead of or in addition to the aforementioned operational feedback for the purpose of helping the operator utilize the device, the controller method 380C may include actively adjusting the operating parameters of the handheld disinfection device, such as the power/intensity of the electromagnetic radiation source. Thus, step 388 may include actively modulating, by the processor, the disinfecting electromagnetic radiation emitted from the electromagnetic radiation source based on the detected operational data (for example, by adjusting the power supplied to the electromagnetic radiation source). In various embodiments, step 388 includes modulating the power level based on the detected distance of the device relative to the surface to be disinfected, thus enabling the user to work on simply maintaining a constant speed and not worry (as much) about trying to keep the device a uniform distance away from the surface/structure as he/she sweeps the device across the surface/structure. This active modulation may be especially useful for structures have uneven surface features, such as seats and/or control panels.

This active modulation may be deployed in conjunction with steps 384 and/or 386 to further improve the disinfecting treatment. For example, the controller may be configured to turn off the electromagnetic radiation source if the accelerometer detects the disinfecting electromagnetic radiation is directed up (e.g., toward the eyes of the operator). This safety feature may be manually overridden to allow the operator to treat the underside of seats with the disinfecting electromagnetic radiation. In various embodiments, the power level, wave-length, or frequency of the electromagnetic radiation source may be actively adjusted based on the distance between the electromagnetic radiation source and the target surface (as detected by the proximity sensor). Further, if the device remains stationary for a period of time, step 388 may include turning off the device to conserve battery and for safety reasons. In various embodiments, step 388 includes determining if the operational data indicates that the disinfection treatment is being performed within certain thresholds, and actively/directly adjusting the electromagnetic radiation source accordingly.

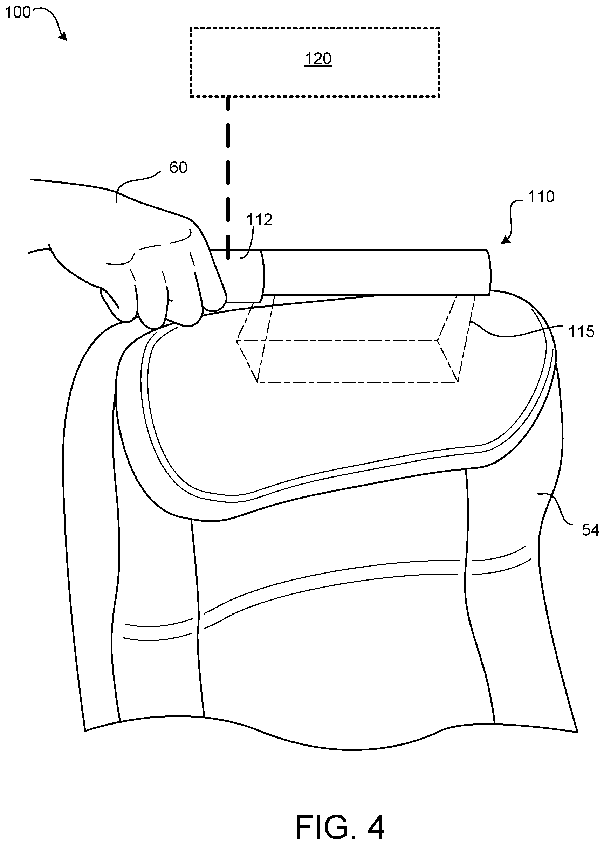

In various embodiments, and with reference to FIG. 4, the handheld disinfection device 110 may also include a targeting module, such as a visible electromagnetic radiation source, coupled to the housing 112 configured to emit visible electromagnetic radiation 115 that is indicative of the disinfecting electromagnetic radiation emitted from the disinfecting electromagnetic radiation source. That is, the visible light 115 may be emitted and oriented to match the emanation and dispersion of the disinfecting electromagnetic radiation (which is invisible to the human eye), thus enabling the operator to visualize the area of the target surface that is receiving the disinfecting radiation.

In various embodiments, and with continued reference to FIG. 4, a disinfection system 100 is provided. The disinfection system 100 may include a handheld disinfection device, such as device 110, and a wearable 120 configured to be worn by the operator 60 and configured to be in electric communication (e.g., wired or wireless) with the handheld disinfection device 110. The wearable 120, which is depicted schematically in FIG. 4, may facilitate communication of the operational feedback from the handheld disinfection device 110 to the operator. That is, the wearable 120 may be configured to deliver the operational feedback to the operator that is pertinent to whether a proper dosage of the disinfecting electromagnetic radiation has been directed to the target surface. As mentioned above, the operational feedback may be dynamic and thus the wearable 120 may be configured to deliver the operational feedback substantially in real-time. In various embodiments, the wearable 120 may include glasses, and thus delivering the operational feedback to the operator via the glasses includes visually displaying the operational feedback to the operator. The glasses may comprise physical filters to enable the operator to see a presence and/or an intensity of the disinfecting electromagnetic radiation. In various embodiments, the glasses may comprise one or more augmented reality screens configured to enable the operator see calculated digital depictions of at least one of a presence and intensity of the disinfecting electromagnetic radiation. In various embodiments, the handheld disinfection device may only be operable by the operator if the wearable is synced/connected thereto. In various embodiments, the glasses may block UV-C light, and thus may provide extra safety to the operator.

In various embodiments, and with reference to FIG. 5, the handheld disinfection device 510 includes housing 512 that includes handle 511 at one end and pivoting head 521 at the opposing end. The head 521 generally includes the electromagnetic radiation source (underside of head 521). The head 521 may also include one or more heat sinks 517 to facilitate heat transfer away from the electromagnetic radiation source(s). The device 510 may also include a display screen 519 that displays the operational data and/or the operational feedback for the operator. The handle 511 may include ergonomic features and may include one or more actuation buttons 513 that control operation of the device. In various embodiments, the handle may extend oblique relative to the main shaft portion of the housing 512. The sensor 516, which is shown schematically in FIG. 5, may be disposed in the shaft of the housing 512 or in the head 521 adjacent the electromagnetic radiation source. Similarly, the controller 518 is also shown schematically in FIG. 5. The controller may be embedded within the housing 512 of the device 510, or the controller 518 may be remote relative to the device and may be electrically connected (via a wired or wireless connection) to the device.

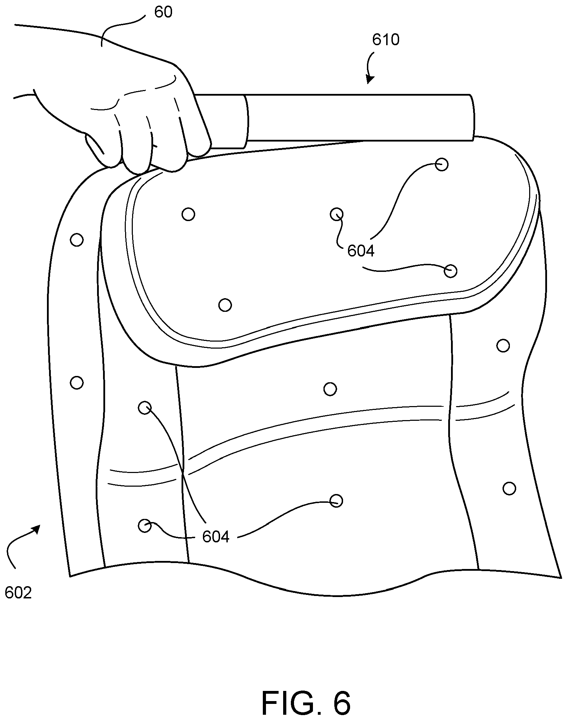

In various embodiments, and with reference to FIG. 6, an article of manufacture is provided that facilitates whether a proper dosage of disinfecting electromagnetic radiation has been received by a structure. That is, the article of manufacture, which may be an aircraft seat 602 of an aircraft, may include structure comprising a surface that is susceptible to indirect contact transmission of pathogens. Article of manufacture may include a material 604 embedded within, coupled to, or integrated with the surface of structure (e.g., the aircraft seat 602), and the material 604 may be configured to provide feedback to an operator pertaining to a disinfecting dosage experienced by the surface of the structure in response to a disinfecting treatment from a handheld disinfecting device 610 manipulated by an operator 60. In various embodiments, the material 604 includes a plurality of nodes distributed across the surface of the aircraft seat 602. The material may passively provide indication to the operator in response to the disinfecting treatment. For example, the plurality of nodes may include photoluminescence units configured to luminesce in response to photoexcitation from the disinfecting electromagnetic radiation. Thus, the photoluminescence of the plurality of nodes may indicate to the operator whether a sufficient dosage of disinfecting electromagnetic radiation has been applied across the structure (i.e., the aircraft seat 602).

Benefits, other advantages, and solutions to problems have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, solutions to problems, and any elements that may cause any benefit, advantage, or solution to occur or become more pronounced are not to be construed as critical, required, or essential features or elements of the disclosure.

The scope of the disclosure is accordingly to be limited by nothing other than the appended claims, in which reference to an element in the singular is not intended to mean "one and only one" unless explicitly so stated, but rather "one or more." It is to be understood that unless specifically stated otherwise, references to "a," "an," and/or "the" may include one or more than one and that reference to an item in the singular may also include the item in the plural. All ranges and ratio limits disclosed herein may be combined.

Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C. Different cross-hatching is used throughout the figures to denote different parts but not necessarily to denote the same or different materials.

The steps recited in any of the method or process descriptions may be executed in any order and are not necessarily limited to the order presented. Furthermore, any reference to singular includes plural embodiments, and any reference to more than one component or step may include a singular embodiment or step. Elements and steps in the figures are illustrated for simplicity and clarity and have not necessarily been rendered according to any particular sequence. For example, steps that may be performed concurrently or in different order are illustrated in the figures to help to improve understanding of embodiments of the present disclosure.

Any reference to attached, fixed, connected or the like may include permanent, removable, temporary, partial, full and/or any other possible attachment option. Additionally, any reference to without contact (or similar phrases) may also include reduced contact or minimal contact. Surface shading lines may be used throughout the figures to denote different parts or areas but not necessarily to denote the same or different materials. In some cases, reference coordinates may be specific to each figure.

Systems, methods and apparatus are provided herein. In the detailed description herein, references to "one embodiment", "an embodiment", "various embodiments", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

Furthermore, no element, component, or method step in the present disclosure is intended to be dedicated to the public regardless of whether the element, component, or method step is explicitly recited in the claims. As used herein, the terms "comprises", "comprising", or any other variation thereof, are intended to cover a non-exclusive inclusion, such that a process, method, article, or apparatus that comprises a list of elements does not include only those elements but may include other elements not expressly listed or inherent to such process, method, article, or apparatus.

## Claims

1. A handheld disinfection device (110), comprising:
a housing (112);
an electromagnetic radiation source (114) affixed to the housing and configured to operably emit disinfecting electromagnetic radiation;
a sensor (116) coupled to the housing; and
a controller (118) coupled to the housing, the controller comprising a processor and a tangible, non-transitory computer-readable storage medium having instructions stored thereon that, in response to execution by the processor, cause the processor to perform operations comprising:
receiving, by the processor, operational data from the sensor pertaining to an operator's manipulation of the handheld disinfection device during a disinfecting treatment.

2. The handheld disinfection device of claim 1, wherein the operations further comprise determining, by the processor, operational feedback based on the operational data, wherein the operational feedback comprises information pertaining to whether a proper dosage of the disinfecting electromagnetic radiation has been directed to a surface of a structure to be disinfected.

3. The handheld disinfection device of claim 2, wherein the operations further comprise providing, by the processor, the operational feedback to the operator.

4. The handheld disinfection device of claim 3, further comprising a display screen configured to display at least one of the operational data and the operational feedback for the operator.

5. The handheld disinfection device of claim 3, wherein the operational feedback is dynamic operational feedback such that determining and providing, by the processor, the dynamic operational feedback to the operator is performed substantially in real-time, and optionally further comprising an indicator coupled to the housing, wherein the indicator is configured to provide at least one of visible, audible, and haptic indications to the operator representative of the dynamic operational feedback.

6. The handheld disinfection device of claim 2, wherein determining, by the processor, the operational feedback comprises tracking, by the processor, dosage of the disinfecting electromagnetic radiation.

7. The handheld disinfection device of claim 6, wherein tracking, by the processor, the dosage of the disinfecting electromagnetic radiation comprises mapping the disinfecting electromagnetic radiation across the surface of the structure that is susceptible to indirect contact transmission of pathogens, and optionally further comprising a wireless connection module affixed to the housing, wherein the operations comprise detecting, by the processor, a dynamic location of the handheld disinfection device.

8. The handheld disinfection device of any preceding claim, wherein the operations further comprise actively modulating, by the processor, the disinfecting electromagnetic radiation emitted from the electromagnetic radiation source based on the operational data.

9. The handheld disinfection device of any preceding claim, wherein the sensor comprises at least one of an accelerometer, a proximity sensor, a location sensor, and an atmospheric sensor, and optionally wherein the atmospheric sensor is configured to detect humidity of air around the handheld disinfection device.

10. The handheld disinfection device of any preceding claim, further comprising a targeting module coupled to the housing and configured to emit visible electromagnetic radiation indicative of the disinfecting electromagnetic radiation emitted from the electromagnetic radiation source.

11. A disinfection system (100), comprising:
a handheld disinfection device (110) comprising an electromagnetic radiation source configured to operably emit disinfecting electromagnetic radiation; and
a wearable (120) configured to be worn by an operator, wherein the wearable is configured to be coupled in electric communication with the handheld disinfection device;
wherein the wearable (120) is configured to provide operational feedback to the operator, wherein the operational feedback comprises information pertaining to whether a proper dosage of the disinfecting electromagnetic radiation has been directed to a surface of a structure to be disinfected.

12. The disinfection system of claim 11, wherein the operational feedback is dynamic and wherein the wearable comprises glasses configured to visually display the operational feedback substantially in real-time to the operator.

13. The disinfection system of claim 12, wherein the glasses comprise physical filters configured to enable the operator to see at least one of a presence and intensity of the disinfecting electromagnetic radiation, or wherein the glasses comprise one or more augmented reality screens configured to enable the operator to see calculated digital depictions of at least one of a presence and intensity of the disinfecting electromagnetic radiation.

14. An article of manufacture, comprising:
a structure (602) comprising a surface susceptible to indirect contact transmission of pathogens; and
a material (604) at least one of embedded within, coupled to, and integrated with the surface of the structure, wherein the material (6) Ois configured to provide feedback to an operator pertaining to a disinfecting dosage experienced by the surface in response to a disinfecting treatment by a disinfection device.

15. The article of manufacture of claim 14, wherein:
the structure is an aircraft seat of an aircraft; and
the material comprises an array of nodes distributed across the surface of the aircraft seat, and optionally wherein the disinfecting treatment comprises disinfecting electromagnetic radiation, wherein the array of nodes comprise photoluminescence units configured to luminesce in response to photoexcitation from the disinfecting treatment.
